# EUROPEAN PATENT SPECIFICATION

(11) **EP 1 204 311 B1**
(45) Date of publication and mention of the grant of the patent: **02.02.2005**
(21) Application number: 00953583.2
(22) Date of filing: 18.08.2000
(51) Int. Cl.: A01K 11/00, A61B 5/07

(54) **MEASUREMENT OF THE ACIDITY IN A GASTRIC SYSTEM OF ANIMALS SUCH AS COWS**
MESSUNG DER MAGENSÄURE VON TIEREN WIE KÜHE
MESURE DE L'ACIDITE DANS LE SYSTEME GASTRIQUE D'ANIMAUX TELS QUE DES VACHES

(30) Priority: 19.08.1999 NL 1012864
(43) Date of publication of application: 15.05.2002
(73) Proprietor: N.V. Nederlandsche Apparatenfabriek NEDAP, 7141 DC Groenlo (NL)
(72) Inventor: VERSTEGE, Albertino, Bernardo, Maria, NL-7121 HM Aalten (NL); ROOSENSCHOON, Pieter, Lieuwe, NL-7104 AC Groenlo (NL)
(74) Representative: Prins, Adrianus Willem, Mr. Ir.
(86) International application number: PCT/NL2000/000575
(87) International publication number: WO 2001/013712

(56) References cited:
- EP-A- 0 646 313
- EP-A- 0 897 662
- WO-A-98/47351
- US-A- 5 279 607

## Description

This invention relates to a method for measuring the acidity in a gastric system of animals such as cows, wherein a pH sensor is introduced into the rumen or a different part of the gastric system of an animal for determining the acidity of a contents of the part of the gastric system in which the sensor is located, while the pH sensor is adapted to enable wireless supply of the measured acidity to a reading device wherein the pH sensor is mounted in a bolus.

Such a method is known from EP-A- 0 646 313. The bolus comprises a power source which may become exhausted over time.

On a farm, animals such as cows are typically fed both roughage and concentrated feeds. In cows, roughage will be subject to rumination, whereby saliva is formed that is transported to the rumen. Due to the saliva thus entering the gastric system of an animal, the degree of acidity (pH) will increase. An increasing value of the pH means that the gastric system is becoming less acidic.

When a cow eats concentrated feed, however, this will not be subject to rumination. The result is that less saliva is transported to the rumen. As a consequence, the acidity will decrease, in other words, the contents of the gastric tract become more acidic.

Research has shown that an optimum acidity for the gastric system of the animal is 5.8 < pH < 7.

On the farm, the animal generally has an unlimited amount of roughage at its disposal, while the concentrated feed is made available to the animal in doses.

The object of the invention is to provide a method which makes it possible to measure the acidity in the gastric system of the animals in a simple and effective manner, which in turn has as a consequence that this information can optionally be used for controlling the amount of concentrates to be supplied to the animals for obtaining an optimum acidity in the gastric system of the animals.

Accordingly, the method according to the invention is characterized in that the pH sensor is received in a case which is provided with an optochemical membrane and that a resonant circuit is provided in the bolus connected to the optochemical membrane so that in use the optochemical membrane generates an electrical signal which signal is a measure for the acidity, said signal is fed through a line connected with the resonant circuit and the acidity is transmitted to a reading device by reading the resonant circuit by means of an interrogation field generated by the reacting device

Because the pH sensor is located in the gastric system of the animal at all times, the acidity can be readily determined at any random moment. If it thus appears that the contents of the gastric system are too acid (i.e. the acidity is too low), less concentrate may be supplied. If it appears that the contents of the gastric system are too basic, supplying more concentrate to the animal might even be considered.

The reading device can be arranged at a suitable position on the farm. In this connection, a milking robot may be considered, so that the acidity is determined at the moment when the animal is milked. It is also possible, however, to set up the reading device at the feeding station, so that the acidity can be determined immediately before the concentrate is furnished to the animal. The amount of concentrate that is administered to the animal can then be adjusted to the instantaneous situation in respect of the acidity of the contents of the gastric system of the animal.

EP-A-0 897 662 also discloses a method according to the preamble of claim 1. However, the bolus disclosed in EP-A-0 897 662 comprises a power source such as a battery for providing electrical power for three years or more.

WO 98/47351 discloses several embodiments of electronic identification devices some of which carry a PH sensor. However, it does not disclose a bolus with a resonant circuit and a PH sensor comprising an optochemical membrane.

The invention will presently be further elucidated with reference to the drawing. In the drawing:

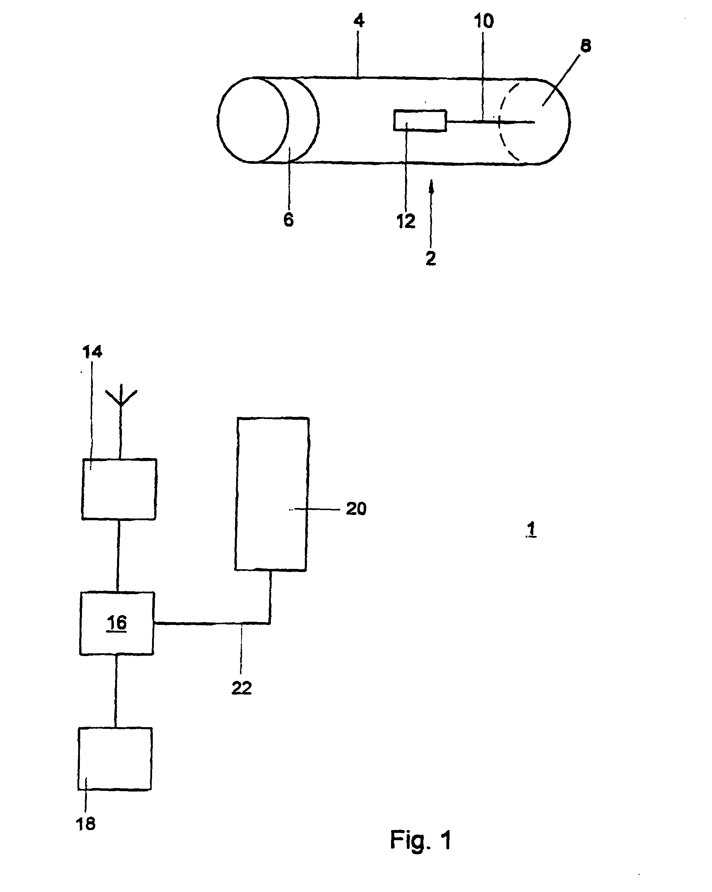

Fig. 1 shows a possible embodiment of a system according to the invention for practicing a method according to the invention.

In Fig. 1, reference numeral 1 designates a system for practicing a method according to the invention. The system comprises a bolus 2, known per se, comprising a glass case 4, which is closed on a first side with a metal cap 6. A second side, located opposite the first side, is closed with an optochemical membrane 8, known per se. The optochemical membrane 8 generates an electric signal on a line 10, which signal is a measure for the acidity of the contents of the environment in which the bolus 2 is located. Further, the bolus 2 includes a resonant circuit 12, known per se, provided with a chip. The resonant circuit 12 can be read out by means of an interrogation field generated by a reading device 14. In the resonant circuit 12, in a manner known per se, an identity code is stored which can be read out by means of the interrogation field. The acidity measured by means of the membrane 8 can also be read out by means of the interrogation field.

In this example, in use, the bolus 2 will be introduced into the rumen or a different part of the gastric system of the animal. In this way, the acidity of the contents of the part of the gastric system where the sensor is located can be measured. The data about the measured acidity in the gastric system of the animal, as well as the identity code of the resonant circuit 12, which identity code has been added to the animal, can be determined by means of the reading device 14. The reading device 14 is connected with the computer 16 for storing and further processing the data about the identity and the acidity as obtained by means of the reading device 14. The computer 16 can also be formed by a farm computer, known per se, in which, further, still other data of the animals are stored. These data can, if desired, be processed in combination. Thus, the computer 16 may be connected with a feeding station 18. The reading device 14 may then be arranged, for instance, in the vicinity of the feeding station 18. When the animal, for instance a cow, goes to the feeding station 18 to feed, the identity of the animal and the acidity of the gastric system of the animal are determined by means of the computer 16 and the reading device 14. Depending on the measured acidity, it can then be regulated how much concentrate will be supplied to the animal by means of the feeding station 18. When it appears that the environment in the gastric system is too acidic (i.e. the acidity expressed in pH is too low) the computer 16 controls the feeding station 18, such that an amount of concentrate less than is average for the animal is made available to the animal. When, conversely, it appears that the environment in the gastric system of the animal is too alkaline (i.e. the acidity expressed in pH is too high), it can even be automatically controlled with the computer 16 that by means of the feeding station 18 an amount of concentrate greater than is average for the animal is made available to the animal in question.

It can also be decided, depending on the measured acidity, to furnish other types of concentrate to the animal.

It is also possible that the reading device 14 is arranged in the vicinity of a milking robot 20 which, in this example, is likewise connected with the computer 16. In that case, the acidity is determined when the animal is milked by means of a milking robot 20. Via line 22 in this example, data about the milk yield can then be likewise applied to the computer 16 for storage and further processing.

The membrane 8, as stated, is included on an outer side of a bolus 2 and closes off the bolus. An advantage of the membrane is that it uses little power and is very durable. The optochemical sensor is then adapted for detecting ions, in this case H⁺ ions. To be considered here is, for instance, microporous teflon filled with a plasticizer in which an ionophore for nitrate is included. Also to be considered here are optochemical pH-sensitive membranes, viz. ETO, NF and CNF, which are all three provided with a protective coating.

The identity of the animal can be stored, together with the measured pH value, in a database of the computer. Preferably, the acidity is measured a plurality of times spread in time by means of the pH sensor, and thus stored in the database. In this way, the course of the pH in time can be further analyzed for each animal by means of a computer. It is also possible that in the bolus a chip is included with memory locations. In this way, information about the course of the acidity in time can be written in the chip. This chip, which, as stated, can be part of the electric circuit 12, can then be read out at the time when the animal is located in the vicinity of the reading device 14. On the basis of the measured pH values, particular relationships can be established. Thus the pH value during the day says something about the fat content of the milk.

The invention is not limited in any way to the embodiments outlined hereinbefore. Thus the bolus 2 can be so adapted as not to provide any identification information about the associated animal. The identification information can then be determined, for instance, on the basis of an ear button of the animal or otherwise. In this way, it is still possible to store the measured acidity together with the associated identity of the animal in the computer 16. Also, on the basis of the acidity, it can be determined whether the animal is perhaps ill. The farmer can then be warned by the farm computer 16. The farmer may then examine the animal more closely. Such variants are each understood to fall within the scope of the invention.

## Claims

1. A method for measuring the acidity in a gastric system of animals such as cows, wherein a pH sensor (8,10,12) is introduced into the rumen or a different part of the gastric system of an animal for determining the acidity of a contents of the part of the gastric system in which the sensor is located, while the pH sensor (8,10,12) is adapted to enable wireless supply of the measured acidity to a reading device (14) wherein the pH sensor is mounted in a bolus (2), **characterized in that** the pH sensor is received in a case (4) which is provided with an optochemical membrane (8) and that a resonant circuit (12) is provided in the bolus (2) connected to the optochemical membrane (8) so that in use the optochemical membrane (8) generates an electrical signal which signal is a measure for the acidity, said signal is fed through a line (10) connected with the resonant circuit (12) and the acidity is transmitted to a reading device (14) by reading the resonant circuit (12) by means of an interrogation field generated by the reading device (14).

2. A method according to claim 1, **characterized in that** the pH sensor (8,10,12) further comprises an electronic memory for recording measured values of the acidity.

3. A method according to claim 1, or 2, **characterized in that**, depending on the acidity in the gastric system of the animal as read out, a particular amount of concentrate is supplied to an animal.

4. A method according to claim 1, **characterized in that** the reading device (14) is arranged adjacent a feeding station (18) for automatically determining how much concentrate is made available to the animal when the animal goes to the feeding station.

5. A method according to any one of the preceding claims, **characterized in that** together with the determination of the acidity, also, in a known manner, the identity of the animal is determined.

6. A method according to claim 5, **characterized in that** the identity and the acidity as determined are stored in a database by means of a computer (16).

7. A method according to claim 6, **characterized in that** the acidity is determined by means of the pH sensor a plurality of times spread in time.

8. A method according to any one of the preceding claims 1-6, **characterized in that** the reading device (14) is set up adjacent a milking robot (20), for determining the acidity when the animal is milked.

## Patentansprüche

1. Verfahren zum Messen der Säure im Magenssystem von Tieren, wie Kühen, wobei ein pH-Sensor (8, 10 , 12) in den Pansen oder einen andere Teil des Magensystems eines Tiers eingeführt wird, um den Säuregehalt des Inhalts des Teils des Magensystems zu bestimmen, in dem sich der Sensor befindet, wobei der pH-Sensor (8, 10, 12) in der Lage ist, eine drahtlose Übertragung des gemessenen Säuregehalts an eine Lesevorrichtung (14) zu ermöglichen, wobei der pH-Sensor in einem Bolus (2) angeordnet ist, **dadurch gekennzeichnet, daß** der pH-Sensor in einem Gehäuse (4) aufgenommen ist, das mit einer optochemischen Membran (8) versehen ist, und daß in dem Bolus (2) eine Resonanzschaltung (12) vorgesehen ist, die mit der optochemischen Membran verbunden ist, so daß die optochemische Membran (8) im Gebrauch ein elektrisches Signal erzeugt, das einen Meßwert des Säuregehalts angibt, wobei das Signal über eine mit der Resonanzschaltung (12) verbundene Leitung (10) geliefert wird und der Säuregehalt an die Lesevorrichtung (14) übertragen wird, indem die Resonanzschaltung (12) mittels eines von der Lesevorrichtung (14) erzeugten Abfragefelds gelesen wird.

2. Verfahren nach Anspruch 1, **dadurch gekennzeichnet, daß** der pH-Sensor (8, 10, 12) ferner einen elektronischen Speicher zum Aufzeichnen gemessener Werte des Säuregehalts aufweist.

3. Verfahren nach Anspruch 1 oder 2, **dadurch gekennzeichnet, daß** je nach dem ausgelesenen Säuregehalt im Magensystem eines Tiers dem Tier eine bestimmte Menge an Konzentrat zugeführt wird.

4. Verfahren nach Anspruch 1, **dadurch gekennzeichnet, daß** die Lesevorrichtung (14) nahe einer Fütterstation (18) angeordnet ist, um automatisch festzustellen, wie viel Konzentrat dem Tier zur Verfügung gestellt wird, wenn sich das Tier zur Fütterstation begibt.

5. Verfahren nach einem der vorhergehenden Ansprüche, **dadurch gekennzeichnet, daß** zusammen mit der Bestimmung des Säuregehaltes auch in an sich bekannter Weise die Identität des Tiers festgestellt wird.

6. Verfahren nach Anspruch 5, **dadurch gekennzeichnet, daß** die festgestellte Identität und der festgestellte Säuregehalt in einer Datenbasis mittels eines Computers (6) gespeichert werden.

7. Verfahren nach Anspruch 6, **dadurch gekennzeichnet, daß** der Säuregehalt mittels des pH-Sensors mehrfach über einen Zeitraum verteilt ermittelt wird.

8. Verfahren nach einem der vorhergehenden Ansprüche 1-6, **dadurch gekennzeichnet, daß** die Lesevorrichtung (14) nahe einem Melkroboter (20) angeordnet ist, um den Säuregehalt festzustellen, wenn das Tier gemolken wird.

## Revendications

1. Procédé permettant de mesurer l'acidité au sein du système gastrique d'animaux tels que des vaches, dans lequel procédé l'on introduit un capteur de pH (8, 10, 12) dans la panse ou une autre partie du système gastrique d'un animal afin de déterminer l'acidité du contenu de la partie du système gastrique où est placé ce capteur, lequel capteur de pH (8, 10, 12), qui est incorporé dans un bol (2), est adapté pour pouvoir envoyer, par transmission sans fil, l'acidité mesurée vers un lecteur (14), **caractérisé en ce que** le capteur de pH est placé dans un boîtier (4) muni d'une membrane optochimique (8) et **en ce que** dans le bol (2) est disposé un circuit oscillant (12) qui est connecté à la membrane optochimique (8), de telle sorte qu'en service, la membrane optochimique (8) génère un signal électrique qui est une mesure de l'acidité, ce signal est transmis par une ligne (10) connectée au circuit oscillant (12), et l'acidité est transmise au lecteur (14), qui lit cette donnée sur le circuit oscillant (12), au moyen d'un champ d'interrogation généré par le lecteur (14).

2. Procédé conforme à la revendication 1, **caractérisé en ce que** le capteur de pH (8, 10, 12) comporte en outre une mémoire électronique qui sert à enregistrer les valeurs d'acidité mesurées.

3. Procédé conforme à la revendication 1 ou 2, **caractérisé en ce que**, en fonction de la valeur lue de l'acidité au sein du système gastrique d'un animal, on fournit à cet animal une certaine quantité d'un concentré.

4. Procédé conforme à la revendication 1, **caractérisé en ce que** le lecteur (14) est installé au voisinage d'un poste d'alimentation (18), pour que soit déterminée automatiquement la quantité de concentré qui doit être mise à disposition de l'animal quand celui-ci se rend au poste d'alimentation.

5. Procédé conforme à l'une des revendications précédentes, **caractérisé en ce que** l'on détermine aussi, en même temps que l'acidité et d'une façon connue, l'identité de l'animal.

6. Procédé conforme à la revendication 5, **caractérisé en ce que** l'identité et l'acidité déterminées sont stockées dans une base de données, au moyen d'un ordinateur (16).

7. Procédé conforme à la revendication 6, **caractérisé en ce que**, au moyen du capteur de pH, on détermine l'acidité à plusieurs moments, espacés dans le temps.

8. Procédé conforme à l'une des revendications précédentes 1 à 6, **caractérisé en ce que** le lecteur (14) est installé au voisinage d'un robot de traite (20), ce qui permet de mesurer l'acidité lors de la traite de l'animal.
